(19) Europäisches Patentamt
European Patent Office
Office européen des brevets

(11) Publication number: 0 221 579 A1

(12) **EUROPEAN PATENT APPLICATION**

(21) Application number: 86201520.3

(51) Int. Cl.[4]: C07D 205/04

(22) Date of filing: 02.09.86

(30) Priority: 30.09.85 GB 8524024

(43) Date of publication of application: 13.05.87 Bulletin 87/20

(84) Designated Contracting States: AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Verbrugge, Pieter Adriaan
Badhuisweg 3
NL-1031 CM Amsterdam(NL)
Inventor: De Waal, Jannetje
Badhuisweg 3
NL-1031 CM Amsterdam(NL)
Inventor: de Lind van Wijngaarden, Gerhard
Badhuisweg 3
NL-1031 CM Amsterdam(NL)
Inventor: van Reijendam, Jan Willem
Badhuisweg 3
NL-1031 CM Amsterdam(NL)

(74) Representative: Hunter, Keith Roger Ian et al
4 York Road
London SE1 7NA(GB)

(54) **A process for the preparation of an azetidine-3-carboxylic acid or salt thereof.**

(57) The invention relates to a process for the preparation of an azetidine-3-carboxylic acid of the formula I:

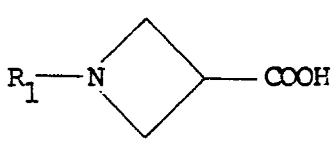

(I)

or a salt thereof, wherein $R_1$ represents a hydrogen atom, a $C_{1-6}$alkyl or $C_{3-8}$ cycloalkyl group or a group phenyl-CH($R_3$)-wherein $R_3$ represents a hydrogen atom, a phenyl or an alkyl group of 1-4 carbon atoms, which process comprises reacting an azetidine of the formula II:

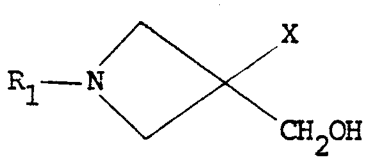

(II)

EP 0 221 579 A1

wherein $R_1$ has the meanings defined above, and X represents a hydrogen atom, a carboxyl or a group, with an alkali metal base at a temperature of from 150 to 300°C in the presence of a catalyst comprising a metal of the Group IIB of the Periodic System and/or a compound of said metal, and if desired converting the obtained azetidine-3-carboxylic acid salt into the corresponding acid; and to novel alkali, alkaline earth and quaternary ammonium salts of such acids.

# A PROCESS FOR THE PREPARATION OF AN AZETIDINE-3-CARBOXYLIC ACID OR SALT THEREOF

The invention relates to a process for the preparation of an acetidine-3-carboxylic acid, unsubstituted or N-substituted, or a salt thereof, and to the work up of such azetidine-3-carboxylic acids and salts. The invention further relates to certain azetidine-3-carboxylic acid salts as novel compounds.

It is disclosed in EP-A-292 65 that azetidine -3-carboxylic acid is a chemical hybridising agent, its mode of action presumably being based on its ability to produce male sterility in plants.

According to the present invention there is provided a process for the preparation of an azetidine-3-carboxylic acid of the formula I:

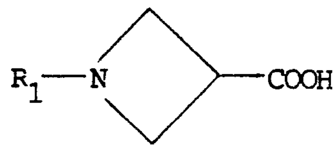

(I)

or a salt thereof, wherein $R_1$ represents a hydrogen atom, a $C_{1-6}$alkyl or $C_{3-8}$ cycloalkyl group or a group phenyl-CH($R_3$)-wherein $R_3$ represents a hydrogen atom, a phenyl or an alkyl group of 1-4 carbon atoms, which process comprises reacting an azetidine of the formula II:

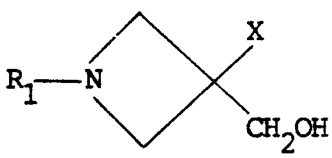

(II)

wherein $R_1$ has the meanings defined above, and X represents a hydrogen atom, a carboxyl or a hydroxymethyl group, with an alkali metal base at a temperature of from 150 to 300°C in the presence of a catalyst comprising a metal of the Group IIB of the Periodic System and/or a compound of said metal, and if desired converting the obtained azetidine-3-carboxylic acid salt into the corresponding acid.

It is known to use zinc or a zinc compound as catalyst in certain dehydrogenation reactions, involving the conversion of an alcohol into a carboxylic acid. For example, it is known from European Patent Specification 31,694 and US Patent Specification 4,241,451 to prepare certain carboxylic acid salts by reaction of alcohols with alkali metal hydroxides in the presence of metallic zinc or a zinc compound. Similarly certain fatty acid salts have been prepared by an alkaline fusion reaction of primary alcohols in the presence of zinc or a zinc compound (see US Patent Specification 3,957,838).

The above-mentioned azetidine-3-carboxylic acids and their salts all contain the azetidine skeleton (or trimethyleneimine skeleton). The four-membered ring (3 carbon atoms plus one nitrogen atom) is under considerable strain. It is generally known from the literature on heterocyclic compounds that the bonds are easily attacked, that the azetidine-ring is fairly easily opened and that azetidines do not, in general, survive drastic hydrolytic treatment. In the preparation of the azetidine compounds a number of other reactions, like polymerization, elimination, fragmentation and solvolysis may compete with ring closure. (See Acheson "An Introduction to the Chemistry of Heterocyclic Compounds", third Ed., INTERSCIENCE, 1976, Chapter II, Paquette "Principles of Modern Heterocylic Chemistry", BENJAMIN, 1968, Chapter III, N.H. Cromwell and B. Phillips "Chemical Reviews", 1979, Vol. 79, No. 4, "The Azetidines. Recent Synthetic Developments.", pages 331-358.) Consequently it is very surprising, indeed quite contrary to expectations, that alkali fusion does not break the ring structure.

It is moreover surprising to find that the end product is predominantly the mono-3-substituted carboxylic acid, whether one starts from the mono-3-hydroxymethyl or the bis-3,3-hydroxymethyl substituted precursor.

In the process according to the invention the reaction temperature is preferably between 175°C and 225°C, especially 180-200°C. The pressure at which the reaction takes place may range from sub-atmospheric pressure up to superatmospheric pressure, e.g. in the order of $10^7$ Pa or even higher. However, the reaction is preferably performed at atmospheric pressure. The catalyst used in the process according to the invention is one or species selected from Group IIB metals and oxides and salts thereof. Preferably the

catalyst is an oxide, or a nitrate, chloride, carbonate, acetate or other salt. The Group IIB metals comprise zinc, cadmium and mercury. Cadmium and, especially, zinc metals or compounds are preferred. Cadmium has higher intrinsic activity but zinc can produce a reaction mixture of lower viscosity. Preferred zinc compounds are zinc oxide, zinc chloride, zinc carbonate, zinc acetate, zinc 2-ethylhexanoate and zinc octoate. The catalysts are employed in a catalytic amount. Generally, from 0.2 to 5.0% by weight - (calculated on the basis of metal) based on the weight of the azetidine derivative may be employed.

The reaction is facilitated by the presence of a water scavenger, suitably calcium oxide. The addition of a water scavenger is especially useful with zinc catalysts which are less active than cadmium catalysts but favoured for other reasons.

In the process according to the invention the one or two hydroxymethyl groups may be formed in situ from their esters, if desired.

The process of the invention may be applied with particular advantage with compounds of formula I in which $R_1$ is a hydrogen atom or a benzyl group, especially the latter, and in which X is a hydrogen atom or a hydroxymethyl group, especially the latter. Thus, a preferred starting compound is N-benzyl-3,3-bis-(hydroxymethyl) azetidine. It is more preferred than the N-benzyl-3-mono-hydroxylmethyl-azetidine because the latter is more difficult to prepare.

The alkali metal base is preferably used in an amount equal to or, more preferably, in excess of the stoichiometric amount, the molar ratio of the alkali metal base to the hydroxy group of the compound of formula I preferably being in the range 1 to 5, especially 1.15 to 2.5. Thus, when the compound of formula I is a diol the molar ratio of the base to the diol compound is preferably from 2 to 10, especially 2.3 to 5. The process according to the invention may be carried out with or without a diluent. The reaction may be carried out in the presence of a high boiling solvent, that is, a solvent having a boiling point higher than the reaction temperature. For example, certain hydrocarbon oils, for example naphthenic hydrocarbon oils, are suitable. Other diluents are for example cyclohexanol, pinacol or decaline. The diluent should be inert under the reaction conditions applied, and should neither polymerize nor degrade.

The diluent should be separable from the reaction mixture after the reaction is finalized or after later working-up steps. In principle all reactants can be introduced into the reaction vessel at the same time or in a short period of time. Also it is possible to add the azetidine compound over a period of time, while the alkali metal base, catalyst and diluent, if present, are already in the reaction vessel. It is also possible to gradually increase the reaction temperature to the desired temperature, when all reactants are already in the reaction vessel.

The water content must be low enough to allow the reaction temperature to reach the above-mentioned range, under the particular reaction pressure employed. At the preferred reaction pressure, namely atmospheric pressure, the water content suitably does not exceed 10% by weight, preferably being less than 5%. It is generally found that if substantially the only water present derives from the alkali metal base employed, and that base contains less than about 15% by weight of water, the reaction proceeds efficiently. Conveniently, pellets of potassium hydroxide generally contain no more than 15% by weight of water.

The alkali metal base used in the process according to the invention is preferably sodium and/or potassium hydroxide, more preferably potassium hydroxide alone. The compound may form a melt with the azetidine compound and with the resulting reaction product, especially when no diluent is present. It has been found that when calcium hydroxide is used instead of the alkali metal base, the reaction did not perform well. It has also been found that when sodium hydroxide is used as a base, the reaction mixture may solidify, but complete conversion of the reaction can be obtained, albeit after longer reaction times.

Aminoacids such as the azetidine acids, and their salts, being very polar and water soluble, are difficult to separate from inorganic salts. Preferred methods for their purification involve precipitation or ion exchange. In one method according to the present invention work-up may be facilitated by contacting a solution of the reaction mixture, suitably in water or an alcohol, with a calcium, magnesium or barium compound, suitably a sulphate or chloride, and preferably magnesium sulphate, barium chloride or, especially, calcium chloride. This technique allows for various possibilities for further work up. For example the group IIA metal salts of azetidine-3-carboxylic acids are soluble in methanol but insoluble in water, whilst their formates precipitate from methanol but remain in solution in water.

For example, when N-benzyl-3,3-bis(hydroxymethyl)azetidine is reacted at a high temperature with an alkali metal hydroxide under the influence of zinc acetate as catalyst, the reaction mixture contains two products, namely the alkali metal salts of N-benzyl-azetidine-3-carboxylic acid and formic acid respectively. Water can be added to the reaction mixture in order to dissolve both solid potassium salts. The zinc

catalyst can be filtered off as zinc oxide. The alkali metal salt of the azetidine-3-carboxylic acid of the formula I wherein $R_1$ has the meanings as defined above together with the formate can then be contacted with, for example, calcium chloride whereby the calcium salt of the azetidine-3-carboxylic acid is formed, and selectively precipitates from solution.

When no water is added, the alkali metal salt of the azetidine-3-carboxylic acid (preferably the N-benzyl substituted azetidine-3-carboxylic acid) together with the formate can be directly taken up in an alcohol, conveniently methanol, ethanol or isopropylalcohol. (This method is especially suitable when no diluent was used in forming the melt.) The group IIA metal compound, such as calcium chloride, dissolved in methanol, is contacted with the solution of both salts and a precipitate consisting of potassium chloride, calcium hydroxide and calcium formate is formed, whilst the calcium salt of the azetidine-3-carboxylic acid remains in solution in methanol.

The group IIA metal salts, such as the calcium salts produced by the techniques described in the preceding two paragraphs, may be converted into the corresponding acids by acidifying them with mineral acids, such as hydrochloric, sulphuric and phosphoric acid, or organic acids. The use of certain acids is of particular benefit because the resulting inorganic salts may precipitate from the solution containing the azetidine acid. For example if phosphoric acid is added to a suspension of the calcium salt of the N-substituted azetidine-3-carboxylic acid in water, a new precipitate comprising calcium phosphate forms and a solution of the N-substituted azetidine-3-carboxylic acid in water is left. The calcium salt of the N-substituted azetidine-3-carboxylic acid may also be reacted with acetic acid, under formation of the N-substituted azetidine-3-carboxylic acid, which is soluble in acetic acid, and with precipitation of the calcium acetate. After separation of the calcium acetate, a solution of the N-substituted azetidine-3-carboxylic acid remains and can be directly treated to obtain azetidine-3-carboxylic acid, acetic acid being advantageously present in a subsequent hydrogenation reaction, which suitably employs palladium on charcoal as catalyst.

Alternatively the calcium salt of N-benzyl-azetidine-3-carboxylic acid in methanol may be treated with a carboxylic acid, for example formic acid or malonic acid whereby the calcium salt of formic or malonic acid is precipitated, because of its insolubility in methanol.

Another method according to the invention involves ion pair extraction, whereby the reaction mixture, dissolved in water, is contacted with an extractant for the azetidine-3-carboyxlic anion, in the presence of a substantially water immiscible organic solvent, to form an aqueous phase containing the unwanted inorganic cations and an organic phase containing at least a part, and preferably substantially all, of the azetidine-3-carboxylic acid anions. The extractant preferably comprises a lipophilic quaternary ammonium ion: the structure of the ammoniun ion may vary but should be such that an ion pair formed by combination of the azetidine-3-carboxylic anion and the ammonium cation is preferentially soluble in the organic phase. Preferably, such an ammonium ion is of the general formula

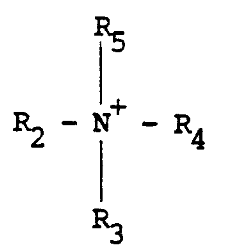

(III)

wherein each of $R_2$, $R_3$, $R_4$ and $R_5$ independently represents an optionally substituted hydrocarbyl group optionally interrupted by one or more hetero atoms, each having from 1 to 22 carbon atoms, and together having at least 7 carbon atoms. Examples of such groups are alkyl and cycloalkyl groups; aryl, aralkyl and alkaryl groups, or like groups comprising a heteroaryl moiety; and groups of formula $-R_6-O-R_6-OR_7$ where each group $R_6$ independently represents an alkylene group, especially ethylene, and $R_7$ represents an alkyl group or, especially, a hydrogen atom. Hetero atoms, where present, are preferably selected from nitrogen, oxygen and sulphur. An example of a suitable extractant is ALIQUAT 366 (trade mark), a trifatty monomethyl quaternary ammonium chloride in which the fatty groups are derived from a mixture of $C_8$ to $C_{10}$ straight chain alkyl groups. If it is desired to perform a single extraction the molar ratio of the ammonium cation to the azetidine anion should be at least one in order to allow the possibility of complete removal of the azetidine anions. However, it may be preferred to conduct several extractions, or a continuous extraction, in which case the molar ratio in a given extraction, or at a given time in a continuous extraction, may be less than one.

Preferably, the azetidine compound is subsequently recovered by the addition to the organic phase of excess aqueous acid, suitably an aqueous organic acid, and preferably formic or acetic acid. The separation may also be effected using an acidic gas, suitably carbon dioxide. The azetidine acid enters the aqueous phase, which is separated. The organic phase may be treated, for recovery of the extractant from the excess acid, with a base, suitably sodium or potassium hydroxide.

The obtained sodium or potassium salt of the N-unsubstituted or N-substituted azetidine-3-carboxylic acids may also be converted to the corresponding azetidine-3-carboxylic acids by ion exchange on a resin column. After the dehydrogenation reaction has taken place, the reaction mixture having been cooled, demineralized water having been added to the reaction mixture and the insoluble compound (zinc oxide) having been filtered off, the salts in water are poured onto an acidic ion exchange column containing activate ion exchange resin.

A number of fractions may be collected with water or ammonia. With, for example, the potassium salt of N-benzyl azetidine-3-carboxylic acid, the neutral and low basic fractions contain N-benzyl azetidine-3-carboxylic acid.

A particularly convenient method of working up a mixture containing the potassium salt of an azetidine-3-carboxylic acid and potassium formate is to treat the mixture with aqueous tartaric and/or oxalic acid, to precipitate potassium bitartrate or bioxalate from the solution.

When a compound of formula I in which R' represents hydrogen is desired, the protecting group R', when present, is removed by catalytic hydrogenation, for example using a platinum or, preferably, palladium catalyst, suitably on a carrier such as charcoal, and preferably with acetic acid present. Hydrogen gas is preferably bubbled through the solution. Alternatively, instead of hydrogen gas, other hydrogen donors may be used, for example, formic acid (J. Chem. Res., (s), 1979, 108-9) or a cycloalkene such as cyclohexene - (Perkins Transactions I, (1977), 490) or 1,4-cyclohexadiene (J. Org. Chem., 43 , (1978), 4194).

The calcium, magnesium, barium and potassium salts of the azetidine-3-carboxylic acids are novel compounds. The sodium salts of the azetidine-3-carboxylic acids are also novel, as isolated compounds. A class of novel compounds according to the invention may thus be defined as azetidine-3-carboxylic acid salts of the formula IV

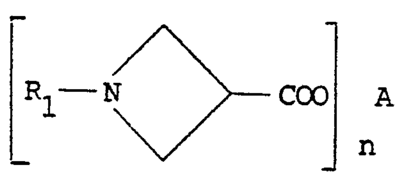

(IV)

wherein n is 2 and A is calcium, magnesium or barium or wherein n is 1 and A is potassium or, when the compounds IV are isolated salts, sodium.

Preferably, $R_1$ represents a hydrogen atom or a group phenyl-$CH(R_3)$-, as defined above, preferably hydrogen or benzyl.

Another class of novel compounds according to the invention are the quaternary ammonium salts containing the cation of formula III and the anion of the compound of formula I as defined above.

The invention will now be further illustrated by reference to the following Examples.

## EXAMPLE 1

In a teflon-reactor (400 ml), rinsed with nitrogen and heated in silicone oil and provided with a magnetic stirring bar and with a condenser connected with a gas volume meter, were introduced:

41,5 g N-benzyl-3,3-bis(hydroxymethyl)azetidine (200 m mol),
39,5 g potassium hydroxide (85% by weight) (600 mmol),
3 g zinc diacetate .$2H_2O$, and
60 ml Ondine 15 (Trade Mark; a naphthenic hydrocarbon oil).

The mixture was heated at 200 °C for 24 hours and generated 14,4 litres of hydrogen. Thereafter the reactor was cooled, the obtained reaction mixture was mixed with 100 ml demineralized water. Solids were removed. Two layers were formed. The lower slightly yellow water layer containing the reaction products was worked up as follows: The water layer was separated and poured on an 2N HCl activated ion exchange column (length 40 cm, diameter 8 cm) containing 1,8 litre Dualite C26TR (Trade Mark) acid ion exchange resin.

21 fractions were collected. of 300 ml with water and ammonia as eluents. In the following Table the fractions are mentioned.

TABLE

| Eluent | Fraction | pH | Colour | Product |
|---|---|---|---|---|
| water | 1,2 | neutral | colourless | - |
| " | 3-8 | acidic | " | - |
| " | 9,10 | neutral | " | - |
| 2N $NH_3$ | 11,12 | neutral | " | - |
| " " | 13-15 | neutral | slightly yellow | 3,2 g |
| " " | 16-18 | pH 8 | brownish | 23,3 g |
| " " | 19,20 | pH 10 | " | 8,1 g |
| " " | 21 | pH 12 | slightly yellow | 0,5 g* |
| " " | 22 | " " | colourless | 0,3 g* |
| " " | 23 | " " | " | 0,3 g* |

Product = N-benzyl azetidine-3-carboxylic acid.
* = Product contaminated with the corresponding monoalcohol ($3-CH_2OH$)

The fractions 13-20 were collected and the fractions were decolourised with carbon. After evaporation of the water under vacuum 34,5 g of a white powder was obtained. Analysis: 96% pure N-benzyl azetidine-3-carboxylic acid. Yield 85,8%. $K^+$ 0,44 %w. $Na^+$ 0,10 % w.

EXAMPLE 2

In a teflon-reactor (400 ml), rinsed with nitrogen and heated in silicone oil and provided with a magnetic stirring rod and with a condenser connected with a gas volume meter, were introduced
41,5 g N-benzyl-3,3-bis(hydroxymethyl)azetidine (200 m mol),
30 g potassium hydroxide (87%) (466 m mol),
3 g zinc diacetate $.2H_2O$.

The mixture was heated at 200 °C for 5 hours and generated 14,3 litre of hydrogen. The reactor was cooled and the obtained reaction mixture was worked up as follows:

The reaction product was directly taken up in 100 ml methanol and the insoluble particles (zinc oxide) were removed. To the light yellow methanol filtrate was added 26,9 g $CaCl_2$ (96% pure) (233 m mol) dissolved in 100 ml methanol, while stirring and keeping the mixture at 60 °C for 1 hour. After the obtained slurry was cooled to 20 °C, the precipitate, consisting of KCl, $Ca(OH)_2$ and calcium formate, was collected on a filter and washed with methanol.

The resulting slightly yellow coloured methanol filtrate contained the calcium salt of N-benzyl azetidine-3-carboxylic acid. The methanol was flashed off and 43,6 g of a white solid residue was obtained.

Physical properties: This calcium salt is soluble in methanol, warm isopropanol, tetrahydrofurane and n-butyl alcohol. It is insoluble in water.

EXAMPLE 3

A. In a teflon-reactor (400 ml), rinsed with nitrogen and heated in silicone oil and provided with a magnetic stirring bar and with a condenser connected with a gas volume meter, were introduced
23,5 g 3,3-bis(hydroxymethyl)azetidine (200 m mol),
30 g potassium hydroxide (87%) (466 m mol),
3 g zinc diacetate .$2H_2O$ and
60 ml Ondine 15 (Trade Mark).
The mixture was heated at 195 °C for 6 hours and generated 14,5 litre of hydrogen. The reactor was cooled and 100 ml demineralized water was added. After removal of the solids (zinc oxide) and formation of the two layers, the lower water layer was separated from the upper hydrocarbon layer. The water layer containing the potassium salt of azetidine-3-carboxylic acid and potassium formate, was evaporated under vacuum. 50 g of a white solid residue was obtained.

B. This residue was taken up in demineralized water and poured on 2N HCl activated Duolite C26TR (Trade Mark) and eluted according to the working up method as described in Example 1: The acidic fractions contained formic acid and 16 g of azeditine-3-carboxylic acid was recovered as a white powder - (purity 96%, determined $^1$H NMR spectroscopy). Yield on intake is 76%.

C. In the same way as described under A, 50 g of a white solid residue was prepared, which was taken up in 150 ml methanol. To this mixture was added 26.9 g $CaCl_2$ 96% (233 m mol) dissolved in 150 ml methanol. After 1 hour stirring at a temperature of 60 °C and standing overnight at room temperature, insoluble compounds were filtered off and the filtrate was flashed. The solid residue was stirred up in iso-propyl alcohol, filtered and dried. 21 g of a white powder was obtained. (I.R. spectrum: 3300 broad large - (OH, NH), 1570 broad large ($COO^-$), 1380, 1160, 775, 730 $cm^{-1}$).

Said white powder was taken up in water of 60 °C and treated with phosphoric acid to pH 5,3 and then with CaO to pH 6,5. After filtration the resulting clear aqueous solution was flashed, leaving 17,3 g of a white solid.

Azetidine-3-carboxylic acid was obtained in 90% purity.

EXAMPLE 4

The same reaction as carried out in Example 2 was performed with the exception that 2 g of cadmium dinitrate ($Cd(NO_3)_2$) was used as a catalyst instead of zinc diacetate. The reaction was extremely fast and after 1,5 hours 14,5 litre of hydrogen was generated. (The orange-brown catalyst could be easily isolated from water or methanol with the aid of 5 g carbon and reused two times before its activity declined.)

The reaction product was worked up as follows: After cooling the obtained reaction product, 100 ml demineralized water was added to dissolve the potassium salts. Insolubles were removed. 26.9 g $CaCl_2$ - (96%) (233 m mol) dissolved in water was added to the aqueous reaction product under vigorous stirring at a temperature of 50 °C. A white voluminous precipitate was formed. After cooling to room temperature the precipitate (containing the calcium salt of N-benzyl azetidine-3-carboxylic acid together with calcium hydroxide) was collected and washed 3 times with water. The still wet precipitate was then suspended in 100 ml demineralized water of 50 °C and treated with phosphoric acid (85%) at pH 5,3 in order to give a new precipitate of different appearance. The pH was immediately adjusted to 7 with 0,5 g CaO. After filtration of the precipitate (containing $Ca_3(PO_4)_2$, $Ca(OH)_2$ and calcium phosphate), the clear slightly yellow filtrate was flashed to dryness under vacuum, leaving N-benzyl azetidine-3-carboxylic acid as a white solid compound (Yield 80%).

EXAMPLE 5

To a strongly alkaline solution of N-benzyl azetidine-3-carboxylic acid (0,05 mol) in 30 ml water an aqueous solution of $MgSO_4$ (0,05 mol in 20 ml water) was added. The resulting mixture was extracted with 50 ml 1-butyl alcohol. After three washes with 20 ml water, the 1-butyl alcohol solution was flashed off leaving 9,5 g of the magnesium salt of N-benzyl azetidine-3-carboxylic acid as a white solid.

The barium salt was prepared as above, using an aqueous solution of $BaCl_2$. The magnesium and the barium salt of N-benzyl azetidine-3-carboxylic acid do not readily form a precipitate from water, but can be easily recovered by extraction as described. IR spectra of the N-benzyl azetidine-3-carboxylic acid salts were measured:

Magnesium salt. I.R. spectrum: 3300 broad large ($OH^-$), 1585 broad large ($COO^-$), 1375, 1365, 1315, 1285, 1275, 1210, 1180, 1140, 1070, 1025, 1000, 965, 910, 880, 845, 820, 760, 740, 700 (all sharp) $cm^{-1}$.
Barium salt. I.R. spectrum: 3300 broad ($OH^-$), 1570 broad ($COO^-$), 1385, 1315, 1285, 1215, 1190, 1154, 1083, 1035, 970, 920, 860, 748, 710, 705 $cm^{-1}$.
Calcium salt. I.R. spectrum: 3420 sharp large ($OH^-$), 2350 broad, 1650 sharp, 1570 large sharp ($COO^-$), 1375, 1360, 1320, 1305, 1280, 1240, 1190, 1142, 1080, 1050, 1020, 1000, 955, 940, 910, 890, 855, 760, 692, 635 (all sharp) $cm^{-1}$).

EXAMPLE 6

In a teflon reactor (400 ml), rinsed with nitrogen and heated in silicone oil and provided with a magnetic stirring rod and with a condenser connected with a gas volume meter, were introduced:
41,5 g N-benzyl-3,3-bis(hydroxymethyl)azetidine (200 mmol)
19,2 g sodium hydroxide (97%) (466 m mol)
3 g zinc diacetate $.2H_2O$.
The mixture was heated at 200 °C for 24 hours and generated 14,4 litres of hydrogen. The reactor was cooled and the reaction product further worked up. The reaction product comprised the sodium salt of N-benzyl azetidine-3-carboxylic acid and sodium formate. The both salts could be separated from each other by making use of the difference in solubility in ethanol. The reaction product was therefore mixed with 250 ml ethanol, the mixture boiled up under stirring and then cooled to room temperature. While the sodium salt of N-benzyl azetidine-3-carboxylic acid went into solution, the sodium formate was removed together with zinc oxide by filtration from the alcoholic solution. The clear ethanol fitrate was then flashed off and the sodium salt of N-benzyl azetidine-3-carboxylic acid precipitated as a white solid compound. 38 g of sodium salt of N-benzyl azetidine-3-carboxylic acid was recovered.
I.R. spectrum-mull-major peaks ($cm^{-1}$)
3400 large broad ($CH^-$), 1650 sharp aromatic, 1580 large broad ($COO^-$), 1405, 1285, 1241, 1215, 1190, 1175, 1155, 1080, 1035, 1010, 960, 910, 860, 780, 745, 700 (all sharp).

EXAMPLE 7

In a teflon-reactor (400 ml), rinsed with nitrogen and heated in silicone oil and provided with a magnetic stirring rod and with a condenser connected with a gas volume meter, were introduced:
41,5 g N-benzyl-3,3-bis(hydroxymethyl)azetidine (200 mmol)
30 g potassium hydroxide (87%) (466 m mol)
3 g zinc diacetate $.2H_2O$.

The mixture was heated at 200 °C for 5 hours and generated 14,3 litre of hydrogen. The reactor was cooled and the obtained reaction mixture worked up as follows. The reaction mixture comprised the potassium salt of N-benzyl azetidine-3-carboxylic acid and potassium formate. The both salts could be separated from each other by making use of the difference in solubility in isopropyl alcohol. The reaction product was therefore mixed with 250 ml isopropyl alcohol, the mixture boiled up under stirring and then cooled to room temperature. While the potassium salt of N-benzyl azetidine-3-carboxylic acid went into solution, the potassium formate was removed together with zinc oxide by filtration from the alcoholic solution. Flashing off the clear solution left 34 g of the potassium salt of N-benzyl azetidine-3-carboxylic acid as a hygroscopic, white solid compound.
I.R. spectrum-mull-major peaks ($cm^{-1}$)
3300 large broad ($OH^-$), 1570 large broad ($COO^-$), 1315, 1300, 1235, 1210, 1190, 1175, 1150, 1075, 1045, 1030, 1000, 964, 927, 915, 905, 880, 855, 815, 780, 760, 740, 715, 695, 625.

EXAMPLES 8

Examples 6 and 7 were repeated.

A. The reaction mixture obtained by the process of Example 6 was worked up in the following way. The reaction mixture was mixed with 250 ml ethanol and 266 mmol formic acid was added to the slurry. After all the sodium formate had been precipitated, it was filtered from the solution. The solvent was then flashed off. 32 g of N-benzyl azetidine-3-carboxylic acid was obtained as a white solid.

B. The reaction mixture obtained by the process of Example 7 was worked up in the following way. The reaction mixture was mixed with 250 ml isopropyl alcohol and 266 mmol formic acid was added to the slurry. After all the potassium formate had been precipitated, it was filtered from the solution. The solvent was then flashed off. 32 g of N-benzyl azetidine-3-carboxylic acid was obtained as a white solid.

EXAMPLE 9

Into a teflon reactor (400ml) were introduced:
83g N-benzyl-3,3-bis (hydroxymethyl)azetidine.
60g potassium hydroxide (87%)
17.1g sodium hydroxide (99%)
1g cadmium dinitrate
The mixture was heated at 200°C for 5 hours and generated 5 litres of hydrogen. The reactor was cooled and the reaction product worked up. 400ml ethanol containing 0.5g phosphoric acid (85%) was added, the mixture heated under stirring and then cooled under stirring. The precipitate, comprising sodium formate and cadmium phosphate, was removed by filtration. The filtrate was worked up, yielding 59.5g of N-benzyl azetidine-3-carboxylic acid, after recrystallization from isopropyl alcohol.

EXAMPLE 10

Into a teflon reactor (400ml) were introduced:
41.5g N-benzyl-3,3-bis(hydroxymethyl)azetidine
30g potassium hydroxide (87%)
0.25g cadmium dinitrate. $4H_2O$
The mixture was heated at 190-200°C for 2 hours. After cooling the reaction product, 100ml of water was added to dissolve the potassium salts. Insolubles (cadmium salts) were removed. 70g tartaric acid - (467mmol) in 70ml water was added. After 2 hours at room temperature the solution was filtered. 86.6g of potassium bitartrate (460mmol) was thus removed. Water and formic acid were flashed off from the remaining solution. Tetrahydrofuran (250ml) was added and the slurry filtered after 2 hours, yielding N-benzyl azetidine-3-carboxylic acid (34.2g). To the acid was added acetic acid (150ml) and 10% palladium on charcoal catalyst (2g). Hydrogen was passed through the mixture at 50-55°C for 22 hours, the mixture was filtered and the solvent was flashed off. Isopropyl alcohol (75ml) was added and after 2 hours the slurry was filtered, then dried, leaving 17g of azetidine-3-carboxylic acid.

EXAMPLE 11

In a teflon reactor (400ml) as described above were introduced:
41.5g N-benzyl-3,3-bis(hydroxymethyl)azetidine
30g potassium hydroxide (87%)
0.3g cadmium dinitrate.4 $H_2O$
The mixture was heated at 190-200°C for 1½ hours. After cooling the reaction product, 100ml of water was added to dissolve the potassium salts. Insolubles (cadmium salts) were removed. 58.7g oxalic acid - (467mmol) in 50 ml water was added. After 2 hours stirring at room temperature the solution was filtered. 58.4g of potassium oxalate (456mmol) was thus removed. Water and formic acid were flashed off from the remaining solution. Acetone (200ml) was added and the slurry filtered after 2 hours, yielding N-benzyl azetidine-3-carboxylic acid (34.3g). To the acid was added acetic acid (100ml) and 10% palladium on charcoal catalyst (2g). Hydrogen was passed through the mixture at 60-65°C for 3½ hours, and the mixture was filtered and the solvent was flashed off. Isopropyl alcohol (75ml) was added and after 18 hours the slurry was filtered, then dried, leaving 17.1g of azetidine-3-carboxylic acid.

EXAMPLE 12

Into a teflon reactor were introduced:
83.5g N-benzyl-3,3-bis(hydroxymethyl)azetidine
60g potassium hydroxide (87%)
20g sodium hydroxide (99%)
20g calcium oxide
6.5g zinc oxide
The mixture was heated at 200°C for 6 hours, with evoluation of 27.5 litres of hydrogen. The reaction mixture was quenched under stirring with 400ml of ethanol containing 6g of phosphoric acid (85%). The solution was cooled, under stirring, to room temperature, filtered, and worked up to yield N-benzyl-azetidine-3-carboxylic acid (56.2g).

EXAMPLE 13

Into a stainless steel reactor with a glass lid and connected via a reflux condensor to a gas meter, was introduced 1 mole of N-benzyl-3,3-bis(hydroxymethyl)azetidine. The reactor was heated in an air bath using a gas flame to 120°C, and 2.3 moles of potassium hydroxide (as 85% pellets) and 0.1 mole of zinc oxide were added. The mixture was heated further with stirring until gas evolution reached approximately 20 litres/hour (this occurred at 190°C). After gas evolution had slowed down the temperature was slowly raised to 210-215°C. The mixture was quenched by slow addition of 200g of water, sufficient to dissolve all reaction products except the catalyst. The time from addition of the potassium hydroxide and zinc oxide to quenching had been 8.7 hours.

High pressure liquid chromatography indicated that the yield of the potassium salt of N-benzyl-azetidine-3-carboxylic acid was 87%.

The reaction mixture was filtered, the filtrate was extracted with methylisobutyl ketone at 50°C, the solution was treated with 50% wt of tartaric acid in water (5mol% excess on intake potassium hydroxide) and cooled to 5°C, and the potassium bitartrate was removed by filtration. The crystals were washed twice with an equal volume of water. The filtrates had a potassium content of less than 30ppm.

The filtrates were concentrated in vacuo, at a temperature of about 50°C, to a concentration of approximately 50%wt of N-benzyl-azetidine-3-carboxylic acid. On cooling to room temperature a small amount of crystals thereof separated out and was filtered off. The remaining solution was taken up in acetic acid and subjected to hydrogenolysis at 50°C, using 1mol% palladium on charcoal as catalyst and hydrogen gas, to produce azetidine-3-carboxylic acid.

EXAMPLE 14

The mixed product of an oxidation reaction as described above, comprising an aqueous solution (320g) of the potassium salt of N-benzyl-azetidine-3-carboxylic acid (250mmol) and 640mmol in total of potassium ions, was purified as follows.

Aliquat 336 (Trade Mark), 331g, was dissolved in SBP 80/110 (trade mark), a petroleum solvent, 331g. A solution of sodium hydroxide (300g) and formic acid (350g) in water (3000g), was added, in 5 portions, with removal of the aqueous phases. The aqueous solution containing the azetidine compound was added and mixed, and the aqueous phase discarded. The organic phase was washed twice with 300g portions of water. The combined three aqueous extracts contained 11.7mmol of N-benzyl-azetidine-3-carboxylic acid. The organic layer was successively back-extracted with four solutions each comprising 11.5g of formic acid in 100g of water. The combined aqueous extracts contained 223mmol of N-benzyl-azetidine-3-carboxylic acid and 0.64mmol of potassium ions.

Excess formic acid may be removed from the remaining organic phase containing the Aliquat 366 by treatment of that phase with a base. The formic acid in the aqueous phase may be flashed off or retained, to serve as a hydrogen donor in a subsequent hydrogenation reaction of the N-benzyl-azetidine-3-carboxylic acid.

## Claims

1. A process for the preparation of an azetidine-3-carboxylic acid of the formula I:

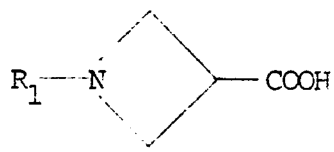

(I)

or a salt thereof, wherein $R_1$ represents a hydrogen atom, a $C_{1-6}$ alkyl or $C_{3-8}$ cycloalkyl group or a group phenyl-CH($R_3$)-wherein $R_3$ represents a hydrogen atom, a phenyl or an alkyl group of 1-4 carbon atoms, which process comprises reacting an azetidine of the formula II:

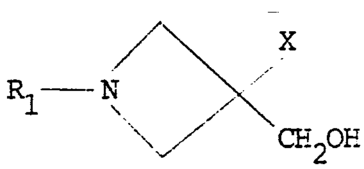

(II)

wherein $R_1$ has the meanings defined above, and X represents a hydrogen atom, a carboxyl or a hydroxymethyl group, with an alkali metal base at a temperature of from 150 to 300°C in the presence of a catalyst comprising a metal of the Group IIB of the Periodic System and/or a compound of said metal, and if desired converting the obtained azetidine-3-carboxylic acid salt into the corresponding acid.

2. A process according to claim 1, wherein the catalyst is one or more species selected from zinc and cadmium and oxides and salts thereof.

3. A process according to any preceding claim 2, wherein the catalyst is zinc oxide and/or zinc acetate.

4. A process according to claim 3, wherein calcium oxide is present.

5. A process according to any preceding claim wherein the alkali metal base is sodium and/or potassium hydroxide.

6. A process according to any preceding claim wherein the azetidine of formula II is N-benzyl-3,3-bis-(hydroxymethyl)-azetidine.

7. A process according to any preceding claim wherein the alkali metal base is used in an amount in excess of the stoichiometric amount calculated on the number of hydroxyl groups in the azetidine.

8. A process according to any preceding claim wherein the reaction is carried out in the presence of a high boiling solvent.

9. A process according to any preceding claim wherein the reaction temperature is in the range 175 °C to 225 °C.

10. A process according to claim 1, substantially as hereinbefore described with particular reference to the Examples.

11. A process according to any of claims 1 to 10 wherein the obtained alkali metal salt of the azetidine-3-carboxylic acid of the formula I:

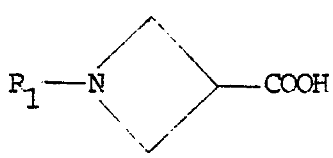

wherein $R_1$ has the meanings as defined in claim 1, together with the obtained formate, is contacted with a calcium, magnesium or barium compound.

12. A process according to claim 11, wherein calcium chloride is used.

13. A process according to claim 11 or 12, wherein the resulting salt of an N-substituted azetidine-3-carboxylic acid is treated with phosphoric acid under precipitation of a phosphate and under formation of a solution of the N-substituted azetidine-3-carboxylic acid, and whereafter the latter carboxylic acid is recovered.

14. A process according to claims 11 or 12, wherein the resulting salt of an N-substituted azetidine-3-carboxylic acid is reacted with acetic acid with precipitation of an acetate and under formation of a solution of the N-substituted azetidine-3-carboxylic acid in acetic acid.

15. A process according to any of claims 1 to 10, wherein a mixture containing the potassium salt of an azetidine-3-carboxylic acid of formula I and potassium formate is treated with aqueous tartaric or oxalic acid.

16. A process according to any of claims 1 to 10 wherein the reaction mixture produced by the process of any of claims 1 to 10 is dissolved in water, contacted with an extractant for the azetidine-3-carboxylic anion, in the presence of a substantially water immiscible organic solvent comprising whereupon the organic phase is separated from the aqueous phase and treated with an aqueous acid to produce a solution of the azetidine acid in aqueous acid.

17. A process according to any preceding claim wherein a resulting N-substituted azetidine-3-carboxylic acid is treated under hydrogenation conditions to obtain azetidine-3-carboxylic acid of the formula IV:

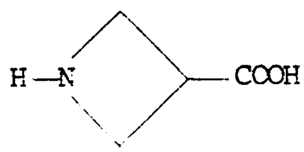

(IV)

18. A process for working up the reaction mixture obtained by the process of the present invention, the working up being substantially as hereinbefore described, with particular reference to the Examples.

19. Compounds whenever prepared by means of a process as claimed in any preceding claim.

20. As novel compounds, azetidine-3-carboxylic acid salts of formula IV,

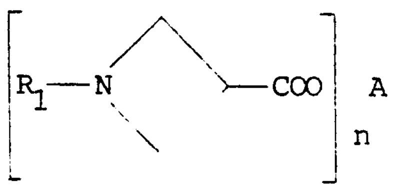

(IV)

wherein n is 2 and A is calcium, magnesium or barium or wherein n is 1 and A potassium, or, (when the compounds IV are isolated solids), sodium.

21. Novel compounds according to claim 20, wherein $R_1$ is a hydrogen atom or a benzyl group.

22. A quaternary ammonium salt of a compound of formula I, wherein the cation is of the general formula

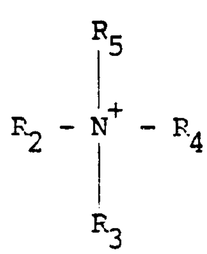

(III)

wherein each of $R_2$, $R_3$, $R^4$ and $R_5$ independently represents an optionally substituted hydrocarbyl group, optionally interrupted by one or more hetero atoms, each having from 1 to 22 carbon atoms, and together having at least 7 carbon atoms.

European Patent Office

# PARTIAL EUROPEAN SEARCH REPORT

which under Rule 45 of the European Patent Convention shall be considered, for the purposes of subsequent proceedings, as the European search report

Application number
EP 86 20 1520

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 114 079 (SHELL)<br>* Claims *<br>--- | 1 | C 07 D 205/04 |
| Y | EP-A-0 140 437 (SHELL)<br>* Page 5, example 4; claims *<br>--- | 1 | |
| P,Y | EP-A-0 165 636 (SHELL)<br>* Claims *<br>--- | 1 | |
| P,A | EP-A-0 168 852 (SHELL)<br>* Pages 6,7 *<br>---------------- | 1 | |
| | | | TECHNICAL FIELDS SEARCHED (Int. Cl.4) |
| | | | C 07 D 205/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9,11-17,19-22

Claims searched incompletely:

Claims not searched: 10,18

Reason for the limitation of the search:

Rule 29.6 of implementing regulations to the Convention on the grant of European Patents.

| Place of search | Date of completion of the search | Examiner |
|---|---|---|
| The Hague | 05-01-1987 | CHOULY |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82